(19) Europäisches Patentamt
European Patent Office
Office européen des brevets

(11) **EP 2 103 288 B1**

(12) **EUROPÄISCHE PATENTSCHRIFT**

(45) Veröffentlichungstag und Bekanntmachung des Hinweises auf die Patenterteilung:
**05.11.2014 Patentblatt 2014/45**

(51) Int Cl.:
***A61F 11/04*** *(2006.01)*

(21) Anmeldenummer: **08007638.3**

(22) Anmeldetag: **18.04.2008**

(54) **Vorrichtung zur auditorischen Stimulation**

Device for auditory stimulation

Dispositif de stimulation auditive

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **20.03.2008 DE 102008015259**

(43) Veröffentlichungstag der Anmeldung:
**23.09.2009 Patentblatt 2009/39**

(60) Teilanmeldung:
**14186204.5**

(73) Patentinhaber:
• **Forschungszentrum Jülich GmbH**
**52425 Jülich (DE)**
• **ANM Adaptive Neuromodulation GmbH**
**52428 Jülich (DE)**
• **Universität zu Köln**
**50923 Köln (DE)**

(72) Erfinder:
• **Tass, Peter**
**81541 München (DE)**

• **Freund, Hans-Joachim**
**40883 Ratingen (DE)**
• **Popovych, Oleksandr**
**52355 Düren (DE)**
• **Barnikol, Birgit Utako**
**52445 Titz (DE)**
• **Niederhauser, Joël**
**4410 Basel (CH)**
• **Roulet, Jean-Christophe**
**2523 Ligniéres/NE (CH)**
• **Schnell, Urban**
**3053 Münchenbuchsee/BE (CH)**

(74) Vertreter: **Manitz, Finsterwald & Partner GbR**
**Martin-Greif-Strasse 1**
**80336 München (DE)**

(56) Entgegenhaltungen:
**WO-A-2004/098690 DE-A1- 10 233 960**
**US-A1- 2005 049 452**

EP 2 103 288 B1

**Beschreibung**

Die Erfindung betrifft eine Vorrichtung zur auditorischen Stimulation.

Bei zahlreichen neurologischen und psychiatrischen Erkrankungen kommt es im Gehirn zu übermäßig starken Synchronisationsvorgängen neuronaler Aktivität, welche die Gehirnfunktion massiv beeinträchtigt. Eine derartige Erkrankung ist der Tinnitus. Tinnitus bezeichnet ein Ohrgeräusch, meist in Form eines hohen Tones, gelegentlich aber auch von klopfendem, pulsierendem oder hämmerndem Charakter. Es handelt sich um eine Volkskrankheit in Form störender Missempfindung, die bei vielen Patienten quälenden Charakter hat. Derzeit verfügbare Therapieverfahren für derartige Krankheiten sind z.B. die Pharmatherapie und die tiefe Hirnstimulation.

Herkömmliche Vorrichtungen und Verfahren zur auditorischen Stimulation sind in den Schriften DE 102 33 960 A1, US 2005/0049452 A1 und WO 2004/098690 A1 beschrieben, wobei die Schrift DE 10233960A1 den nächstliegenden Stand der Technik offenbart.

Vor diesem Hintergrund werden Vorrichtungen gemäß den Ansprüchen 1 und 16 angegeben. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1          eine schematische Darstellung einer Vorrichtung 100 gemäß einem Ausführungsbeispiel;
Fig. 2          eine Darstellung von Sinusschwingungen mit den Frequenzen $f_1$, $f_2$, $f_3$ und $f_4$;
Fig. 3          eine Darstellung einer mit einer Rechteckfunktion amplitudenmodulierten Sinusschwingung;
Fig. 4          eine schematische Darstellung einer Vorrichtung 400 gemäß einem weiteren Ausführungsbeispiel;
Fig. 5          eine schematische Darstellung einer Vorrichtung 500 gemäß einem weiteren Ausführungsbeispiel;
Fig. 6          eine schematische Darstellung einer Vorrichtung 600 gemäß einem weiteren Ausführungsbeispiel;
Fig. 7          eine schematische Darstellung einer Vorrichtung 700 gemäß einem weiteren Ausführungsbeispiel;
Fig. 8          eine schematische Darstellung einer Vorrichtung 800 gemäß einem weiteren Ausführungsbeispiel;
Fig. 9          eine schematische Darstellung einer Vorrichtung 900 gemäß einem weiteren Ausführungsbeispiel;
Fig. 10         eine schematische Darstellung eines auditorischen Stimulationsverfahrens;
Fig. 11         eine schematische Darstellung eines weiteren auditorischen Stimulationsverfahrens;
Fig. 12         eine schematische Darstellung eines weiteren auditorischen Stimulationsverfahrens;
Fig. 13         eine schematische Darstellung eines weiteren auditorischen Stimulationsverfahrens;
Fig. 14         eine schematische Darstellung eines weiteren auditorischen Stimulationsverfahrens; und
Fig. 15A und 15B     schematische Darstellung der Generierung von Modulationssignalen.

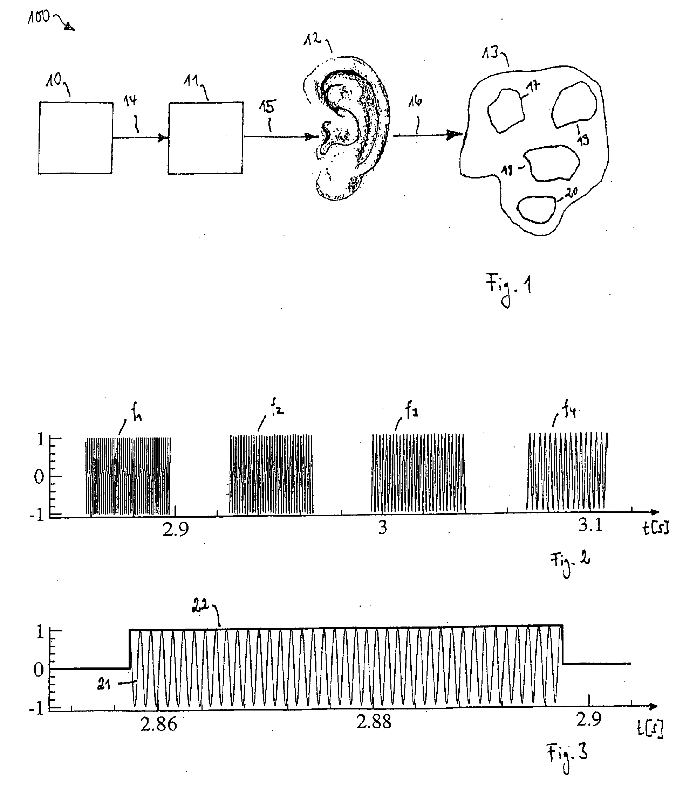

In Fig. 1 ist schematisch eine Vorrichtung 100 dargestellt, die aus einer Steuereinheit 10 und einer mit der Steuereinheit 10 verbundenen Stimulationseinheit 11 besteht. In Fig. 1 sind ferner ein Ohr 12 eines Patienten sowie der auditorische Cortex 13 im Gehirn des Patienten schematisch dargestellt.

Während des Betriebs der Vorrichtung 100 wird die Stimulationseinheit 11 von der Steuereinheit 10 mittels eines oder mehrerer Steuersignale 14 angesteuert und die Stimulationseinheit 11 erzeugt anhand des Steuersignals 14 ein oder mehrere akustische Stimulationssignale 15. Das Frequenzspektrum des akustischen Stimulationssignals 15 kann ganz oder teilweise im für den Menschen hörbaren Bereich liegen. Das akustische Stimulationssignal 15 wird von dem Patienten über ein oder beide Ohren 12 aufgenommen und über den oder die Hörnerven 16 an Neuronenpopulationen im Gehirn weitergeleitet. Das akustische Stimulationssignal 15 ist derart ausgestaltet, dass es Neuronenpopulationen im auditorischen Cortex 13 stimuliert. Im Frequenzspektrum des akustischen Stimulationssignals 15 sind zumindest eine erste Frequenz $f_1$ und eine zweite Frequenz $f_2$ vorhanden. Das akustische Stimulationssignal 15 kann ferner noch weitere Frequenzen oder Frequenzgemische enthalten, in dem in Fig. 1 gezeigten Ausführungsbeispiel sind dies eine dritte Frequenz $f_3$ und eine vierte Frequenz $f_4$.

Die Vorrichtung 100 kann insbesondere zur Behandlung von neurologischen oder psychiatrischen Erkrankungen, wie z.B. Tinnitus, Migräne, Kopfschmerzen unterschiedlicher Form und Genese (z.B. Cluster-Kopfschmerz), Trigeminusneuralgie, Schlafstörungen, Neuralgien und Kopfschmerzen bei Neuroborelliose, Aufmerksamkeits-Defizit-Syndrom (ADS, Attention Deficit Syndrome), Aufmerksamkeits-Defizit-Hyperaktivitäts-Syndrom (ADHS, Attention Deficit Hyperactivity Syndrome), Neurosen, Zwangserkrankungen, Depressionen, Manie, Schizophrenie, Tumoren, Herzrhythmusstörungen, Suchterkrankungen, Bruxismus (nächtliches Zähneknirschen) oder Essstörungen, aber auch anderen Krankheiten verwendet werden.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d.h. die beteiligten Neuronen feuern übermäßig synchron. Hinzu kommt, dass die kranke Neuronenpopulation eine oszillatorische neuronale Aktivität aufweist, d.h. die Neuronen feuern rhythmisch. Bei den oben genannten Krankheiten liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen qualitativ anders, z.B. auf unkontrollierte Weise.

Das von der Stimulationseinheit 11 erzeugte akustische Stimulationssignal 15 wird im Innenohr in Nervenimpulse umgesetzt und über den Hörnerv 16 zu dem auditorischen Cortex 13 weitergeleitet. Durch die tonotope Anordnung des auditorischen Cortex 13 wird bei der akustischen Stimulation des Innenohres mit einer bestimmten Frequenz ein bestimmter Teil des auditorischen Cortex 13 aktiviert. Die tonotope Anordnung des auditorischen Cortex ist z.B. in den folgenden Artikeln beschrieben: "Tonotopic organization of the human auditory cortex as detected by BOLD-FMRI" von D. Bilecen, K. Scheffler, N. Schmid, K. Tschopp und J. Seelig (erschienen in Hearing Research 126, 1998, Seiten 19 bis 27), "Representation of lateralization and tonotopy in primary versus secondary human auditory cortex" von D. R. M. Langers, W. H. Backes und P. van Dijk (erschienen in NeuroImage 34, 2007, Seiten 264 bis 273) und "Reorganization of auditory cortex in tinnitus" von W. Mühlnickel, T. Elbert, E. Taub und H. Flor (erschienen in Proc. Natl. Acad. Sci. USA 95, 1998, Seiten 10340 bis 10343).

In dem Beispiel gemäß Fig. 1 ist das akustische Stimulationssignal 15 so ausgestaltet, dass mit ihm eine Neuronenpopulation des auditorischen Cortex 13 mit einer krankhaft synchronen und oszillatorischen Aktivität stimuliert wird. Diese Neuronenpopulation lässt sich vor Beginn der Stimulation zumindest gedanklich in verschiedene Subpopulationen untergliedern, u.a. in die in Fig. 1 gezeigten Subpopulationen 17, 18, 19 und 20. Vor Beginn der Stimulation feuern die Neuronen aller Subpopulationen 17 bis 20 weitgehend synchron und im Mittel mit der gleichen pathologischen Frequenz. Aufgrund der tonotopen Organisation des auditorischen Cortex 13 werden mittels der ersten Frequenz $f_1$ die erste Subpopulation 17, mittels der zweiten Frequenz $f_2$ die zweite Subpopulation 18, mittels der dritten Frequenz $f_3$ die dritte Subpopulation 19 und mittels der vierten Frequenz $f_4$ die vierte Subpopulation 20 stimuliert. Die Stimulation mit dem akustischen Stimulationssignal 15 bewirkt in den jeweiligen Subpopulationen 17 bis 20 ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen bestimmten Phasenwert, z.B. 0°, gesetzt. Somit wird die Phase der neuronalen Aktivität der krankhaften Subpopulationen 17 bis 20 mittels einer gezielten Stimulation kontrolliert.

Aufgrund der tonotopen Anordnung des auditorischen Cortex 13 sowie der Mehrzahl von Frequenzen $f_1$ bis $f_4$, die in dem akustischen Stimulationssignal 15 enthalten sind, ist es möglich, die krankhafte Neuronenpopulation an den unterschiedlichen Stellen 17 bis 20 gezielt zu stimulieren. Dies ermöglicht es, die Phase der neuronalen Aktivität der krankhaften Neuronenpopulation an den unterschiedlichen Stimulationsstellen 17 bis 20 zu unterschiedlichen Zeitpunkten zurückzusetzen, indem die Frequenzen $f_1$ bis $f_4$ zu unterschiedlichen Zeitpunkten appliziert werden. Im Ergebnis wird dadurch die krankhafte Neuronenpopulation, deren Neuronen zuvor synchron und mit gleicher Frequenz und Phase aktiv waren, in die Subpopulationen 17 bis 20 aufgespalten. Innerhalb jeder der Subpopulationen 17 bis 20 sind die Neuronen weiterhin synchron und feuern auch weiterhin im Mittel mit derselben pathologischen Frequenz, aber jede der Subpopulationen 17 bis 20 weist bezüglich ihrer neuronalen Aktivität die Phase auf, die ihr durch den Stimulationsreiz mit der zugehörigen Frequenz $f_1$ bis $f_4$ aufgezwungen wurde.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d.h. die komplette Desynchronisation, ist somit nach der Applikation des akustischen Stimulationssignals 15 über die Stimulationseinheit 11 nicht sofort vorhanden, sondern stellt sich meist innerhalb weniger Perioden oder gar in weniger als einer Periode der pathologischen Aktivität ein.

Bei der vorstehend beschriebenen Art der Stimulation wird die letztlich gewünschte Desynchronisation durch die krankhaft gesteigerte Interaktion zwischen den Neuronen erst ermöglicht. Hierbei wird ein Selbstorganisationsprozess ausgenutzt, der für die krankhafte Synchronisation verantwortlich ist. Derselbe bewirkt, dass auf eine Aufteilung einer Gesamtpopulation in Subpopulationen mit unterschiedlichen Phasen eine Desynchronisation folgt.

Darüber hinaus kann durch die Stimulation mit der Vorrichtung 100 eine Neuorganisation der Konnektivität der gestörten neuronalen Netzwerke erzielt werden, sodass lang anhaltende, die akustische Stimulation deutlich überdauernde therapeutische Effekte bewirkt werden können.

Um den auditorischen Cortex 13 an unterschiedlichen Stellen, z.B. den in Fig. 1 gezeigten Stellen bzw. Subpopulationen 17 bis 20, fokal zu stimulieren, müssen reine Töne der zugehörigen Frequenzen $f_1$, $f_2$, $f_3$ und $f_4$ verabreicht werden. Infolge der tonotopen Anordnung des auditorischen Cortex 13 werden unterschiedliche Teile des Gehirns durch die gleichzeitige Verabreichung der zugehörigen unterschiedlichen reinen Töne $f_1$ bis $f_4$, d.h. durch die Superposition verschiedener Sinusschwingungen stimuliert. Sollen die vier unterschiedlichen Orte 17 bis 20 z.B. zu unterschiedlichen Zeiten gereizt werden, werden die vier verschiedenen Frequenzen $f_1$ bis $f_4$ zu den jeweiligen Zeiten appliziert. Beispielhaft ist dies in Fig. 2 gezeigt. Hier werden Sinusschwingungen mit den Frequenzen $f_1$ = 1000 Hz, $f_2$ = 800 Hz, $f_3$ = 600 Hz und $f_4$ = 400 Hz sukzessive und pulsförmig appliziert, was zu einer sukzessiven fokalen Reizung an den vier verschiedenen Orten 17 bis 20 des auditorischen Cortex 13 führt. Die Stärke der durch die jeweilige Sinusschwingung erzeugten Reizung des jeweiligen Areals im auditorischen Cortex 13 entspricht der Amplitude der jeweiligen Sinusschwingung.

Die Generierung der in Fig. 2 gezeigten pulsförmigen Sinusschwingungen ist in Fig. 3 beispielhaft dargestellt. Dort wird eine Sinusschwingung 21 mit einer Rechteckfunktion 22, die beispielsweise die Werte 0 oder 1 annehmen kann, multipliziert. Zu den Zeitpunkten, zu denen die Rechteckfunktion 22 den Wert 0 hat, ist der zugehörige Reiz abgeschaltet und während der Zeit, in der die Rechteckfunktion 22 gleich 1 ist, ist der Reiz angeschaltet. Anstelle der Rechteckfunktion 22 kann die Sinusschwingung 21 mit einer beliebigen anderen Funktion multipliziert werden. Im Ergebnis entspricht diese Multiplikation einer Amplitudenmodulation der Sinusschwingung 21.

Anstelle der vorstehend beschriebenen Sinusschwingungen können auch oszillierende Signale mit einer anderen Signalform, wie z.B. Rechtecksignale, die mit der entsprechenden Grundfrequenz oszillieren, zur Generierung des akustischen Stimulationssignals 15 herangezogen werden.

Sofern statt einer fokalen Reizung eine weniger fokale Reizung durchgeführt werden soll, die größere Teile des auditorischen Cortex 13 aktiviert, so werden Frequenzgemische anstelle von einzelnen Frequenzen, beispielsweise pulsförmig appliziert. Mittels eines Frequenzgemisches in den Grenzen zwischen einer unteren Frequenz $f^{unten}$ und einer höheren Frequenz $f^{oben}$ werden all die Teile des auditorischen Cortex 13 gereizt, die durch die Frequenzen zwischen $f^{unten}$ und $f^{oben}$ aufgrund der tonotopen Anordnung stimuliert werden. Sollen z.B. vier unterschiedliche größere Bereiche des auditorischen Cortex 13 zu unterschiedlichen Zeiten stimuliert werden, so werden die vier zugehörigen Frequenzgemische mit den Grenzen $f_j^{unten}$ und $f_j^{oben}$ (j = 1, 2, 3, 4) zu den gewünschten Zeiten appliziert.

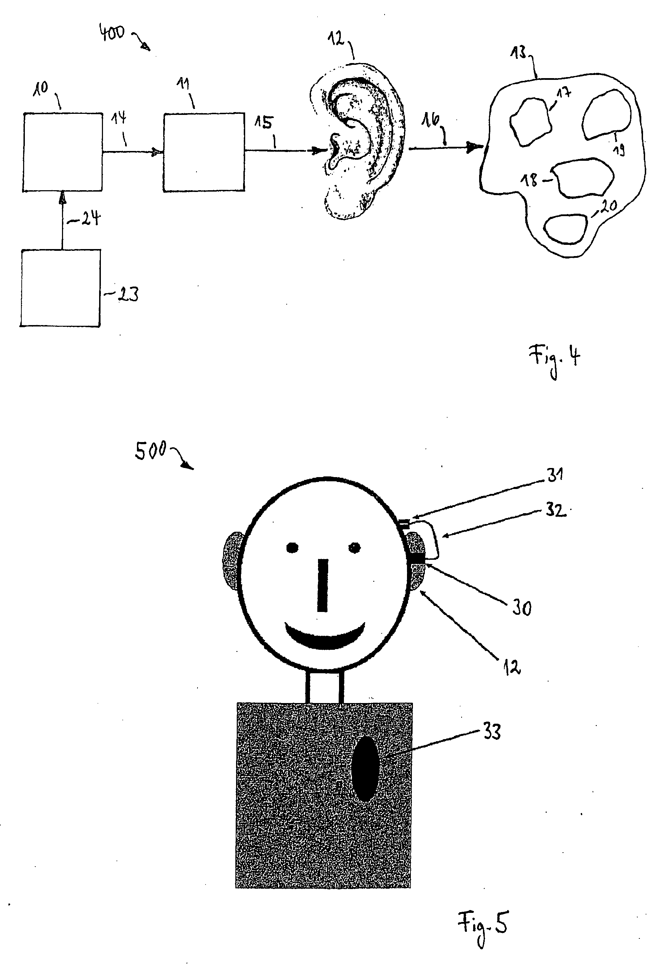

Die Vorrichtung 100 kann beispielsweise in einem sogenannten "open loop"-Modus betrieben werden, bei welchem die Steuereinheit 10 die Stimulationseinheit 11 derart ansteuert, dass diese vorgegebene akustische Stimulationssignale 15 während einer bestimmten Stimulationszeit (z.B. während mehrerer Stunden) erzeugt. Des Weiteren kann die Vorrichtung 100 auch zu einer in Fig. 4 gezeigten Vorrichtung 400 weitergebildet werden, welche ein sogenanntes "closed loop"-System darstellt. Die Vorrichtung 400 enthält zusätzlich zu den aus Fig. 1 bekannten Komponenten noch eine Messeinheit 23, welche ein oder mehrere am Patienten aufgenommene Messsignale 24 bereitstellt und diese an die Steuereinheit 10 weiterleitet. Es kann vorgesehen sein, dass die Steuereinheit 10 anhand der von der Messeinheit 23 aufgenommenen Messsignale 24 die Stimulationseinheit 11 ansteuert. Bei der Messeinheit 23 kann es sich um nicht-invasive Sensoren handeln, wie z.B. Elektroenzephalographie (EEG)-Elektroden, Magnetenzephalographie (MEG)-Sensoren, Akzelerometer, Elektromyographie (EMG)-Elektroden und Sensoren zur Bestimmung von Blutdruck, Atmung oder Hautleitwiderstand. Ferner kann die Messeinheit 23 in Form eines oder mehrerer Sensoren in den Körper des Patienten implantiert sein. Als invasive Sensoren können beispielsweise epikortikale, intrakortikale oder subkutane Elektroden dienen. Insbesondere kann mittels der Messeinheit 23 die physiologische Aktivität in dem stimulierten Zielgebiet oder einem damit verbundenen Gebiet gemessen werden.

Hinsichtlich des Zusammenwirkens der Steuereinheit 10 mit der Messeinheit 23 sind verschiedene Ausgestaltungen denkbar. Beispielsweise kann von der Steuereinheit 10 eine bedarfsgesteuerte Stimulation durchgeführt werden. Hierzu detektiert die Steuereinheit 10 anhand der von der Messeinheit 23 aufgenommenen Messsignale 24 das Vorhandensein und/oder die Ausprägung eines oder mehrerer krankhafter Merkmale. Beispielsweise kann die Amplitude oder der Betrag der neuronalen Aktivität gemessen werden und mit einem vorgegebenen Schwellwert verglichen werden. Die Steuereinheit 10 kann so ausgestaltet sein, dass eine Stimulation eines oder mehrerer Zielgebiete im auditorischen Cortex gestartet wird, sobald der vorgegebene Schwellwert überschritten wird. Ferner können von der Steuereinheit 10 anhand der Ausprägung der krankhaften Merkmale Parameter der akustischen Stimulationssignale 15, wie beispielsweise die Amplituden der jeweiligen Sinusschwingungen oder die Pausen zwischen Stimulationssequenzen, eingestellt werden. Z.B. können ein oder mehrere Schwellwerte vorgegeben werden, und bei einem Überschreiten oder Unterschreiten der Amplitude oder des Betrags der Messsignale 24 über bzw. unter einen bestimmten Schwellwert variiert die Steuereinheit 10 einen bestimmten Parameter des akustischen Stimulationssignals 15, wie z.B. die Amplitude einer oder mehrerer Sinusschwingungen, aus denen das akustische Stimulationssignal 15 zusammengesetzt wird.

Des Weiteren kann vorgesehen sein, dass die von der Messeinheit 23 aufgenommenen Messsignale 24 direkt oder gegebenenfalls nach einem oder mehreren Verarbeitungsschritten in akustische Stimulationssignale 15 umgesetzt werden und von der Stimulationseinheit 11 appliziert werden. Beispielsweise können die Messsignale 24 verstärkt und gegebenenfalls nach mathematischer Verrechnung (z.B. nach Mischung der Messsignale 24) mit einer Zeitverzögerung und linearen und/oder nichtlinearen Verrechnungsschritten als Steuersignale in den Steuereingang der Stimulationseinheit 11 eingespeist werden. Der Verrechnungsmodus wird hierbei so gewählt, dass der krankhaften neuronalen Aktivität entgegengewirkt wird und die akustischen Stimulationssignale 15 mit abnehmender krankhafter neuronaler Aktivität ebenfalls verschwinden oder zumindest deutlich in ihrer Stärke (Amplitude) reduziert werden.

In Fig. 5 ist schematisch eine Vorrichtung 500 dargestellt, die eine Weiterbildung der in Fig. 1 gezeigten Vorrichtung 100 darstellt. Keine Komponente der Vorrichtung 500 muss implantiert werden, sodass sich die gesamte Vorrichtung 500 außerhalb des Körpers des Patienten befindet. Außerdem verwendet die Vorrichtung 500 kein von einem Sensor gemessenes Signal zur bedarfsgesteuerten Variation der Stimulation. Als Stimulationseinheit 11 wird bei der Vorrichtung 500 ein Schallgenerator (Lautsprecher) verwendet, der in einen Ohrstöpsel 30 eingefasst ist. Der Ohrstöpsel 30 wird in den äußeren Gehörgang eines Ohrs 12 des Patienten eingefügt und mit oder ohne Bügel bzw. einer anderen geeigneten mechanischen Hilfe am Ohr 12 befestigt. Die Steuereinheit 10, welche den Schallgenerator ansteuert, sowie eine Batterie oder ein Akku zur Stromversorgung der elektrischen Komponenten der Vorrichtung 500 können in einer oder mehreren separaten Einheiten 31 untergebracht sein. Die Einheit 31 kann mittels einer mechanischen Halterung, z.B. einem Bügel, mit dem Ohrstöpsel 30 verbunden sein. Ein Verbindungskabel 32 verbindet den Ohrstöpsel 30 mit der Steuereinheit 10 bzw. der Batterie.

Alternativ kann statt des Ohrstöpsels 30 auch ein Kopfhörer verwendet werden, der die Steuereinheit 10 und die Batterie enthält. Die Vorrichtung 500 kann vom Patienten mittels einer Bedieneinheit (z.B. Anschaltknopf und/oder Drehregler) angeschaltet werden, die entweder an der Einheit 31 oder direkt am Ohrstöpsel 30 angebracht ist. Mit dem Drehregler kann z.B. die maximale Stimulationsstärke eingestellt werden. Zusätzlich zu den vorstehend genannten Komponenten kann die Vorrichtung 500 über ein Steuermedium 33 verfügen, welches beispielsweise telemetrisch (z. B. über Funk) oder über ein Verbindungskabel mit der Steuereinheit 10 verbunden ist. Im Falle einer Verbindung über ein Kabel können Steckverbindungen zur Konnektierung bzw. Dekonnektierung verwendet werden.

Ferner kann die Vorrichtung 500 auch über ein weiteres, z.B. vom Arzt zu bedienendes Steuermedium (nicht dargestellt) verfügen, welches telemetrisch oder über ein Verbindungskabel mit der Steuereinheit 10 verbunden ist. Im Falle einer Verbindung über ein Kabel können Steckverbindungen zur Konnektierung bzw. Dekonnektierung verwendet werden.

Des Weiteren können ein oder mehrere Sensoren, z.B. EEG-Elektroden oder ein Akzelerometer, zur Registrierung und/oder Dokumentation des Stimulationserfolgs und zur Untersuchung durch den Arzt vorgesehen sein.

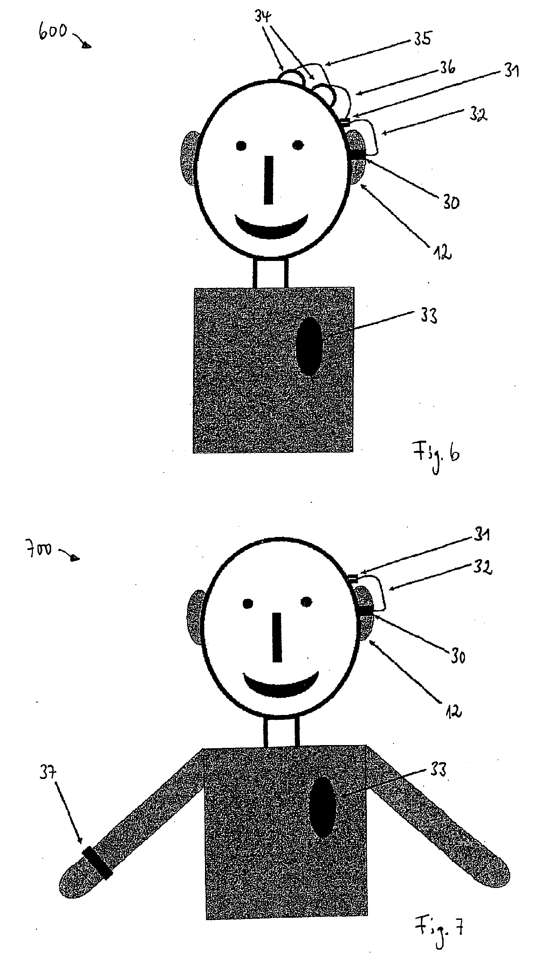

In den Fig. 6 bis 9 sind schematisch Vorrichtungen 600, 700, 800 und 900 als Weiterbildungen der Vorrichtung 400 dargestellt. Die Vorrichtungen 600 bis 900 verfügen jeweils über eine Messeinheit 23, mit der sich eine Bedarfssteuerung und/oder eine Rückkopplung (Feedback) der Messsignale 24 in die Stimulationseinheit 11 durchführen lässt. Hierbei stellen die Vorrichtungen 600 und 700 nicht-invasive Varianten dar, während die Vorrichtungen 800 und 900 zum Teil in den Körper des Patienten implantiert werden. Wie die Vorrichtung 500 umfassen die Vorrichtungen 600 bis 900 einen Ohrstöpsel 30 oder einen Kopfhörer mit einem Schallgenerator.

Die in Fig. 6 dargestellte Vorrichtung 600 verfügt neben den oben beschriebenen Komponenten der Vorrichtung 500 über epikutane, d.h. auf der Haut des Patienten befestigte EEG-Elektroden 34, die über Verbindungskabel 35, 36 mit der Steuereinheit 10 in der Einheit 31 verbunden sind. Die Steuereinheit 10 verstärkt die mittels der EEG-Elektroden 34 gemessene Potentialdifferenz und verwendet diese nach einer optionalen linearen oder nicht-linearen Verrechnung zur Ansteuerung des Schallgenerators in dem Ohrstöpsel 30. Als Alternative zu den Verbindungskabeln 35, 36 können die EEG-Elektroden 34 auch schnurlos, d.h. telemetrisch mit der Steuereinheit 10 verbunden sein. Dies hat den Vorteil, dass der Patient nicht durch Verbindungskabel behindert wird und z.B. an Hindernissen hängen bleiben kann.

Die in Fig. 7 dargestellte Vorrichtung 700 weist anstelle einer EEG-Elektrode ein Akzelerometer (Beschleunigungsmesser) 37 als Messeinheit auf. Der Akzelerometer 37 ist an einer krankheitsbedingt zitternden Gliedmaße des Patienten, z.B. in der Art einer Uhr, befestigt. Die von dem Akzelerometer 37 aufgenommenen Beschleunigungssignale werden in der Steuereinheit 10 verstärkt und nach einer optionalen linearen oder nicht-linearen Verrechnung zur Ansteuerung des Schallgenerators in dem Ohrstöpsel 30 verwendet. Der Akzelerometer 37 kann telemetrisch oder über ein Verbindungskabel mit der Steuereinheit 10 verbunden sein.

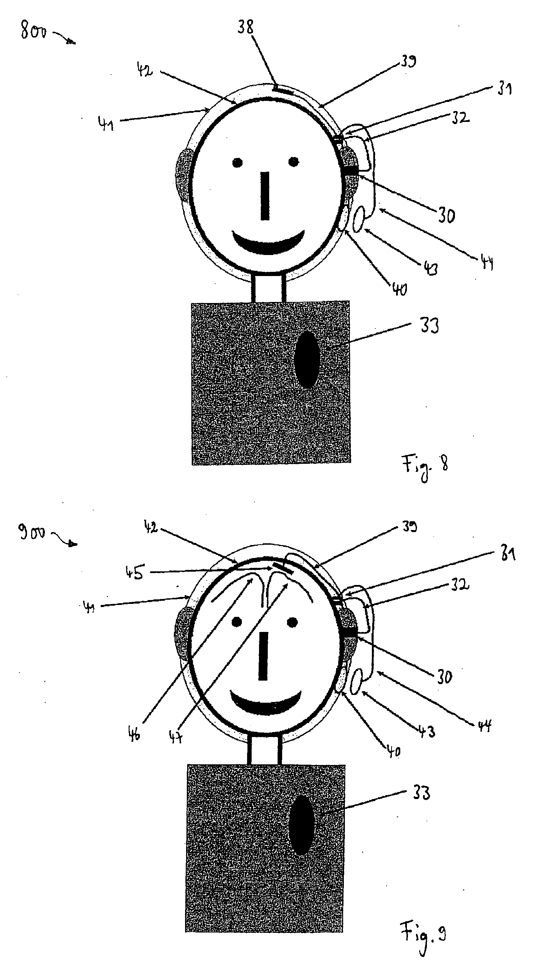

In Fig. 8 ist eine invasive Variante gezeigt. In dem dargestellten Ausführungsbeispiel umfasst die Vorrichtung 800 eine oder mehrere subkutan implantierte Elektroden 38 als Messeinheit, ein Verbindungskabel 39 und eine Sende- und Empfangseinheit 40, die im Körper des Patienten unter der Kopfhaut 41 und außerhalb des knöchernen Schädels 42 implantiert sind. Außerhalb des Körpers des Patienten befinden sich eine Sende- und Empfangseinheit 43, die über ein Verbindungskabel 44 mit der Einheit 31 und der darin befindlichen Steuereinheit 10 verbunden ist. Über die Sende- und Empfangseinheiten 40 und 43, die beispielsweise jeweils als Spule implementiert sind und zwischen denen drahtlos sowie bidirektional Signale als auch elektrische Leistung übertragen werden können, werden die von der Elektrode 38 aufgenommenen Messsignale 24 an die Steuereinheit 10 weitergeleitet. In der Steuereinheit 10 werden die von der Elektrode 38 gemessenen Potentialdifferenzen verstärkt und nach einer optionalen linearen oder nicht-linearen Verrechnung zur Ansteuerung des in den Ohrstöpsel 30 integrierten Schallgenerators herangezogen.

Ein weitere invasive Variante ist schematisch in Fig. 9 dargestellt. Bei der dort gezeigten Vorrichtung 900 dienen eine oder mehrere epikortikal implantierte Elektroden 45 als Messeinheit. "Epikortikal" bedeutet "auf der Hirnrinde gelegen"; zur Illustration ist in Fig. 9 die Hirnrinde 46, 47 beider Hemispheren schematisch gezeigt. Die Steuereinheit 10 verstärkt die mittels der epikortikal implantierten Elektrode 45 gemessene Potentialdifferenz und verwendet diese nach einer optionalen linearen oder nicht-linearen Verrechnung zur Ansteuerung des Schallgenerators in dem Ohrstöpsel 30.

Die in Fig. 9 gezeigte epikortikale Elektrode 45 kann beispielsweise auch durch eine intrakortikale Elektrode ersetzt werden (nicht dargestellt).

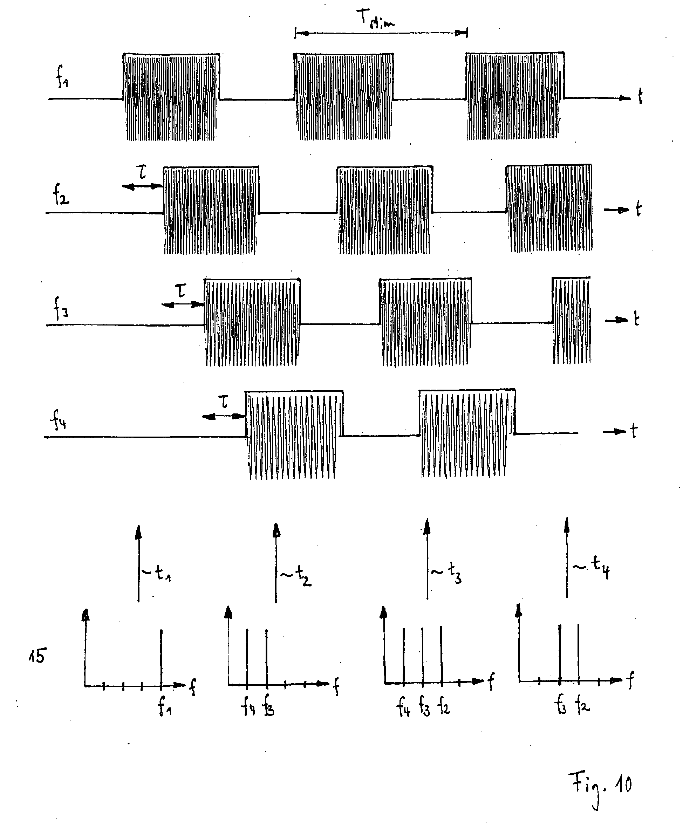

Die von den verschieden ausgestalteten Messeinheiten 23, d.h. den EEG-Elektroden 34, dem Akzelerometer 37 bzw. den Elektroden 38 oder 45, aufgenommenen Messsignale können, wie weiter unten noch ausführlicher beschrieben wird, zur Feedbacksteuerung genutzt werden und als Ansteuersignale in den Schallgenerator eingespeist werden. Alternativ kann anhand der Messsignale 24 eine Bedarfssteuerung durchgeführt werden. Bei einer Stimulation, die auf ein Zurücksetzen der neuronalen Phasen von Neuronensubpopulationen abzielt, können bestimmte Parameter des Stimulationsverfahrens, wie z.B. die Stimulationsstärke oder die Stimulationsdauer, anhand der Messsignale 24 eingestellt werden. Diese Art der Bedarfssteuerung wird weiter unten im Zusammenhang mit den Fig. 10 bis 12 noch näher erläutert.

Anhand der vier Frequenzen $f_1$ bis $f_4$ soll nachfolgend beispielhaft erläutert werden, wie durch zeitversetztes Zurücksetzen der Phasen der neuronalen Aktivität von Subpopulationen einer krankhaft synchronen und oszillatorischen Neuronenpopulation eine Desynchronisation der gesamten Neuronenpopulation erzielt werden kann. Die vier Frequenzen $f_1$ bis $f_4$ sind lediglich beispielhaft zu verstehen, d.h. es kann eine beliebige andere Zahl von Frequenzen oder Frequenzgemischen zu Stimulationszwecken eingesetzt werden. Die Frequenzen $f_1$ bis $f_4$ sind so ausgewählt worden, dass mit ihnen jeweils bestimmte Bereiche 17 bis 20 des auditorischen Cortex 13 stimuliert werden. Dies ermöglicht die oben beschriebene Aufspaltung einer krankhaften Neuronenpopulation in Subpopulationen 17 bis 20. Damit die Subpopulationen 17 bis 20 nach der Stimulation unterschiedliche Phasen aufweisen, können die Frequenzen $f_1$ bis $f_4$ beispielsweise zeitversetzt appliziert werden.

Ein für die oben beschriebenen Zwecke geeignetes Stimulationsverfahren, das beispielsweise mit einer der Vorrichtungen 100 bis 900 durchgeführt werden kann, ist in Fig. 10 schematisch dargestellt. In Fig. 10 sind in den oberen vier Zeilen untereinander vier Sinusschwingungen mit den Frequenzen $f_1$, $f_2$, $f_3$ bzw. $f_4$ gegen die Zeit t aufgetragen. Aus den dargestellten Sinusschwingungen wird das akustische Stimulationssignal 15 gebildet. Zur Erzeugung von pulsförmigen Sinusschwingungen sind die vier Sinusschwingungen mit Rechteckfunktionen multipliziert worden. Jeder Sinusschwingungspuls wiederholt sich periodisch mit einer Frequenz $f_{stim}$. Die Frequenz $f_{stim} = 1/T_{stim}$ liegt im Bereich von 1 bis 30 Hz, insbesondere im Bereich von 5 bis 20 Hz. Derartige Sequenzen von pulsförmigen Sinusschwingungen sind, wenn sie als akustische Stimulationssignale 15 appliziert werden, geeignet, die neuronale Phase der jeweils stimulierten krankhaften Neuronen-Subpopulation 17, 18, 19 bzw. 20 zurückzusetzen. Der Phasenreset ergibt sich dabei nicht notwendigerweise bereits nach einem oder wenigen Pulsen, sondern es können eine gewisse Anzahl der in Fig. 10 gezeigten Sinusschwingungspulse erforderlich sein, um die neuronale Phase der jeweiligen Subpopulation 17, 18, 19 bzw. 20 zurückzusetzen.

Die Frequenz $f_{stim}$ kann beispielsweise im Bereich der mittleren Frequenz der krankhaft rhythmischen Aktivität des Ziel-Netzwerks liegen. Bei neurologischen und psychiatrischen Erkrankungen liegt die mittlere Frequenz typischerweise im Bereich von 1 bis 30 Hz. Beim Tinnitus findet sich z.B. im Frequenzbereich von 1,5 bis 4 Hz übermäßig synchrone neuronale Aktivität. Hierbei ist zu beachten, dass die Frequenz, mit welcher die krankhaften Neuronen synchron feuern, üblicherweise nicht konstant ist, sondern durchaus Variationen aufweisen kann und darüber hinaus bei jedem Patienten individuelle Abweichungen zeigt.

Zur Ermittlung der Frequenz $f_{stim}$ kann beispielsweise die mittlere Peakfrequenz der krankhaften rhythmischen Aktivität des Patienten bestimmt werden. Diese Peakfrequenz kann dann als Stimulationsfrequenz $f_{stim}$ verwendet werden oder auch variiert werden, beispielsweise in einem Bereich von $f_{stim}$ - 3 Hz bis $f_{stim}$ + 3 Hz. Alternativ kann aber auch ohne vorherige Messung eine Frequenz $f_{stim}$ im Bereich von 1 bis 30 Hz gewählt werden und diese beispielsweise während der Stimulation variiert werden, bis die Frequenz $f_{stim}$ gefunden wird, mit der sich die besten Stimulationserfolge erzielen lassen. Als weitere Alternative kann für die Stimulationsfrequenz $f_{stim}$ ein für die jeweilige Krankheit bekannter Literaturwert herangezogen werden. Eventuell kann dieser Wert noch variiert werden, bis beispielsweise optimale Stimulationsergebnisse erzielt werden.

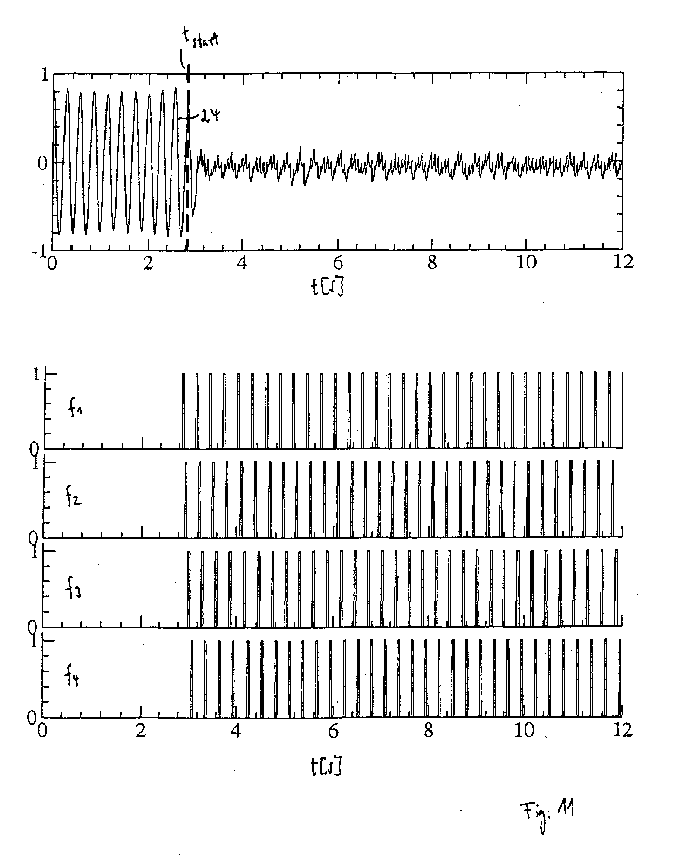

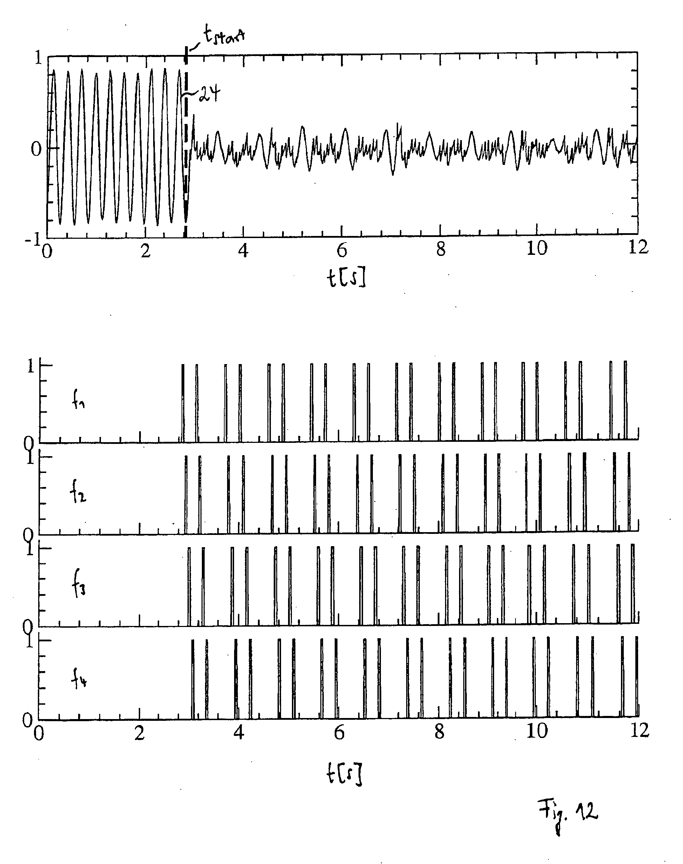

Die Dauer eines Sinusschwingungspulses, d.h. die Zeitspanne, in dem in der vorliegenden Ausgestaltung die Rechteckfunktion den Wert 1 annimmt, kann beispielsweise $T_{stim}/2$ betragen. In diesem Fall sind die Zeitspanne, während der die jeweilige Frequenz zur Stimulation beiträgt, und die nachfolgende Stimulationspause gleich lang. Es ist aber auch möglich andere Stimulationsdauern zu wählen, beispielsweise im Bereich von $T_{stim}/2 - T_{stim}/10$ bis $T_{stim}/2 + T_{stim}/10$. Auch andere Stimulationsdauern sind möglich, z.B. beträgt die Stimulationsdauer bei den in den Fig. 11 und 12 gezeigten Stimulationen $T_{stim}/4$. Die Stimulationsdauern können beispielsweise experimentell bestimmt werden.

Gemäß der in Fig. 10 gezeigten Ausgestaltung erfolgt die Verabreichung der einzelnen Frequenzen $f_1$ bis $f_4$ mit einer zeitlichen Verzögerung zwischen den einzelnen Frequenzen $f_1$ bis $f_4$. Beispielsweise kann der Beginn zeitlich aufeinander folgender und unterschiedliche Frequenzen aufweisender Pulse um eine Zeit $\tau$ verschoben sein.

Im Fall von N Frequenzen, die zur Stimulation eingesetzt werden, kann die zeitliche Verzögerung $\tau$ zwischen jeweils zwei aufeinander folgenden Pulsen beispielsweise im Bereich eines N-tels der Periode $T_{stim} = 1/f_{stim}$ liegen. In dem in Fig. 10 gezeigten Ausführungsbeispiel (N = 4) beträgt die zeitliche Verzögerung $\tau$ dementsprechend $T_{stim}/4$. Von der Vorgabe, dass die zeitliche Verzögerung $\tau$ zwischen jeweils zwei aufeinander folgenden Sinusschwingungspulsen $T_{stim}/N$ beträgt, kann bis zu einem gewissen Grad abgewichen werden. Beispielsweise kann von dem Wert $T_{stim}/N$ für die zeitliche Verzögerung $\tau$ um bis zu $\pm 10\%$, $\pm 20\%$ oder $\pm 30\%$ abgewichen werden. Bei derartigen Abweichung wurden noch Stimulationserfolge erzielt, d.h. es konnte noch ein desynchronisierender Effekt beobachtet werden.

Aus den periodischen Sinusschwingungspulsen mit den Frequenzen $f_1$ bis $f_4$ wird durch Superposition das akustische Stimulationssignal 15 gebildet. Die einzelnen Sinusschwingungspulse können dabei beispielsweise linear oder nicht-linear miteinander kombiniert werden. Dies bedeutet, dass die Sinusschwingungen der einzelnen Frequenzen $f_1$ bis $f_4$ nicht notwendigerweise mit den gleichen Amplituden zu dem akustischen Stimulationssignal 15 kombiniert werden müssen. In der untersten Zeile von Fig. 10 ist beispielhaft das Frequenzspektrum des akustischen Stimulationssignals 15 zu vier verschiedenen Zeitpunkten $t_1$, $t_2$, $t_3$ und $t_4$ dargestellt. Die dort gezeigten Frequenzspektren, insbesondere die Höhe und Form der Frequenzpeaks, sind lediglich beispielhaft zu verstehen und können auch völlig unterschiedliche Formen aufweisen. Im Einzelnen lassen sich den dargestellten Frequenzspektren die folgenden Aussagen entnehmen: Zum Zeitpunkt $t_1$ tritt lediglich die Frequenz $f_1$ in dem akustischen Stimulationssignal 15 auf. Zum Zeitpunkt $t_2$ sind dies die Frequenzen $f_3$ sowie $f_4$, zum Zeitpunkt $t_3$ die Frequenzen $f_2$ bis $f_4$ und zum Zeitpunkt $t_4$ die Frequenzen $f_2$ sowie $f_3$.

Gemäß einer alternativen Ausgestaltung werden statt der Frequenzen $f_1$ bis $f_4$ vier Frequenzgemische mit den Grenzen $f_j^{unten}$ und $f_j^{oben}$ (j = 1, 2, 3, 4) verwendet. In einem Frequenzgemisch j kann eine beliebige Anzahl von Frequenzen im Bereich von $f_j^{unten}$ bis $f_j^{oben}$ vorliegen.

Gemäß einer weiteren alternativen Ausgestaltung werden anstelle der Rechteckfunktionen andere Funktionen zur Amplitudenmodulation der Sinusschwingungen eingesetzt werden, z.B. Sinushalbwellen, deren Frequenz kleiner als $f_1$ bis $f_4$ ist. Ferner ist es beispielsweise denkbar, dass dreieckförmige Pulse als Modulationsfunktionen eingesetzt werden. Ein solcher Puls kann eine sprungförmigen Onset (von 0 auf 1) aufweisen und danach einen Abfall auf 0, wobei der Abfall beispielsweise durch eine lineare oder exponentielle Funktion gegeben sein kann. Durch die Modulationsfunktion wird letztlich die Form der Einhüllenden der einzelnen Pulse bestimmt.

In Fig. 11 ist die bereits in Fig. 10 gezeigte Stimulation über einen längeren Zeitraum hinweg dargestellt. Die einzelnen Sinusschwingungen mit den Frequenzen $f_1$ = 1000 Hz, $f_2$ = 800 Hz, $f_3$ = 600 Hz und $f_4$ = 400 Hz sind in Fig. 11 nicht gezeigt, sondern nur die jeweiligen rechteckförmigen Einhüllenden. Ferner ist in Fig. 11 ein beispielsweise von der Messeinheit 23 aufgenommenes Messsignal 24 dargestellt, das die neuronale Aktivität im auditorischen Cortex vor und während der Stimulation wiedergibt. Die Periode $T_{stim}$ beträgt vorliegend 1/(3,5 Hz) = 0,29 s.

Die Stimulation wird zum Zeitpunkt $t_{start}$ gestartet. Dem Messsignal 24, das in dem vorliegenden Beispiel bandpassgefiltert worden ist, ist zu entnehmen, dass die Neuronen im auditorischen Cortex vor Beginn der Stimulation eine synchrone und oszillatorische Aktivität aufweisen. Kurz nach Beginn der Stimulation wird die krankhaft synchrone neuronale Aktivität im Zielgebiet bereits unterdrückt.

Von dem in den Fig. 10 und 11 gezeigten streng periodischen Stimulationsmuster kann auf unterschiedliche Art und Weise abgewichen werden. Beispielsweise braucht die zeitliche Verzögerung $\tau$ zwischen zwei aufeinander folgenden Sinusschwingungspulsen nicht notwendigerweise stets gleich groß zu sein. Es kann vorgesehen sein, dass die zeitlichen Abstände zwischen den einzelnen Sinusschwingungspulsen unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

Des Weiteren können während der Applikation des akustischen Stimulationssignals 15 Pausen vorgesehen werden, während denen keine Stimulation erfolgt. Die Pausen können beliebig lang gewählt werden und insbesondere ein ganzzahliges Vielfaches der Periode $T_{stim}$ betragen. Die Pausen können nach einer beliebigen Anzahl von Stimulationen eingehalten werden. Z.B. kann eine Stimulation während N aufeinander folgender Perioden der Länge $T_{stim}$ durchgeführt werden und anschließend eine Stimulationspause während M Perioden der Länge $T_{stim}$ eingehalten werden, wobei N und M kleine ganze Zahlen sind, z.B. im Bereich von 1 bis 15. Dieses Schema kann entweder periodisch fortgesetzt werden oder stochastisch und/oder deterministisch, z.B. chaotisch, modifiziert werden.

In Fig. 12 ist eine derartige Stimulation gezeigt. Hier gelten N = 2 und M = 1. Ansonsten entspricht die Stimulation der in Fig. 11 gezeigten Stimulation.

Eine weitere Möglichkeit, von dem in Fig. 10 gezeigten streng periodischen Stimulationsmuster abzuweichen, besteht darin, die zeitlichen Abstände zwischen aufeinander folgenden Pulsen einer Frequenz $f_j$ oder eines Frequenzgemische mit den Grenzen $f_j^{unten}$ und $f_j^{oben}$ (j = 1, 2, 3, 4) stochastisch oder deterministisch oder gemischt stochastisch-deterministisch zu variieren.

Des Weiteren kann pro Periode $T_{stim}$ (oder in anderen Zeitschritten) die Reihenfolge, in welcher die beteiligten Frequenzen $f_j$ oder Frequenzgemische mit den Grenzen $f_j^{unten}$ und $f_j^{oben}$ appliziert werden, variiert werden. Diese Variation kann stochastisch oder deterministisch oder gemischt stochastisch-deterministisch erfolgen.

Ferner kann pro Periode $T_{stim}$ (oder in einem anderen Zeitintervall) nur eine bestimmte Anzahl der Frequenzen $f_j$ oder Frequenzgemische mit den Grenzen $f_j^{unten}$ und $f_j^{oben}$ appliziert werden und die an der Stimulation beteiligten Frequenzen $f_j$ oder Frequenzgemische mit den Grenzen $f_j^{unten}$ und $f_j^{oben}$ können in jedem Zeitintervall variiert werden. Auch diese Variation kann stochastisch oder deterministisch oder gemischt stochastisch-deterministisch erfolgen.

Die vorstehend beschriebenen Stimulationssignale bewirken, dass die Phase der neuronalen Aktivität der krankhaften Neuronenpopulation an den unterschiedlichen Stimulationsstellen zu unterschiedlichen Zeitpunkten zurückgesetzt wird. Dadurch wird die krankhafte Neuronenpopulation, deren Neuronen zuvor synchron und mit gleicher Frequenz und Phase aktiv waren, in mehrere Subpopulationen aufgespalten, was letztlich zu einer Desynchronisation führt.

Alle vorstehend beschriebenen Stimulationsformen können auch in einem "closed loop"-Modus durchgeführt werden. Das Zurücksetzen der Phasen der einzelnen Subpopulation kann beispielsweise mit einer Bedarfssteuerung verknüpft werden. Z.B. kann ein Schwellwert vorgegeben werden, und bei einem Über- bzw. Unterschreiten der Amplitude des Messsignals 24 über bzw. unter den Schwellwert kann die Stimulation gestartet bzw. unterbrochen werden. Ferner können anhand der Amplitude des Messsignals 24, das beispielsweise während Stimulationspausen aufgenommen werden kann, bestimmte Stimulationsparameter, wie z.B. die Amplitude/Stärke der Stimulationssignale oder die Dauer der Stimulation, eingestellt werden. Darüber hinaus ist es möglich, anhand der mittleren Frequenz des (evtl. bandpassgefilterten) Messsignals 24 die Frequenz $f_{stim}$ einzustellen oder nachzujustieren.

Außerdem ist es denkbar, dass die Stimulation durch den Patienten gestartet wird, beispielsweise durch eine telemetrische Aktivierung. In diesem Fall kann der Patient die Stimulation für einen vorgegebenen Zeitraum von z.B. 5 Minuten aktivieren oder der Patient kann die Stimulation selbsttätig starten und beenden.

Im Folgenden werden weitere Ausgestaltungen der "closed loop"-Stimulation beschrieben, die beispielsweise mittels der in Fig. 4 gezeigten Vorrichtung 400 oder einer der Vorrichtungen 600 bis 900 durchgeführt werden können. Wie bereits weiter oben beschrieben wurde, kann das von der Messeinheit 23 aufgenommene Messsignale 24 dazu verwendet werden, ein Steuersignal 14 zu generieren, mit dem die Stimulationseinheit 11 angesteuert wird. Dabei kann das Messsignal 24 entweder direkt oder gegebenenfalls nach einem oder mehreren Verarbeitungsschritten in das akustische Stimulationssignal 15 umgesetzt werden und von der Stimulationseinheit 11 appliziert werden. Der Verrechnungsmodus kann hierbei so gewählt werden, dass der krankhaften neuronalen Aktivität entgegengewirkt wird und das akustische Stimulationssignal 15 mit abnehmender krankhafter neuronaler Aktivität ebenfalls verschwindet oder zumindest deutlich in seiner Stärke reduziert wird.

Bevor das Messsignal 24 in den Steuereingang der Stimulationseinheit 11 eingespeist wird, kann das Messsignal 24 linear oder nicht-linear verarbeitet werden. Beispielsweise kann das Messsignal 24 gefiltert und/oder verstärkt und/oder mit einer Zeitverzögerung beaufschlagt werden und/oder mit einem anderen Messsignal 24 gemischt werden. Ferner kann mit dem Messsignal 24 oder dem verarbeiteten Messsignal 24 die Amplitude einer Sinusschwingung mit einer Frequenz im hörbaren Bereich moduliert werden und die amplitudenmodulierte Sinusschwingung kann danach mittels des Schallgenerators als akustisches Stimulationssignal 15 oder als Teil davon appliziert werden.

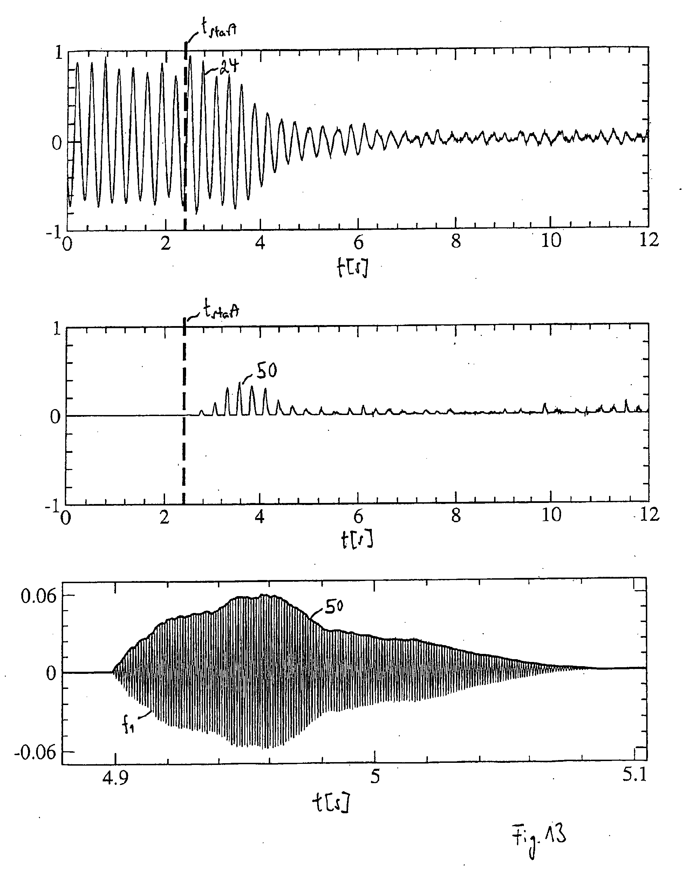

Zur Amplitudenmodulation einer Sinusschwingung oder einer anderen oszillierenden Schwingung muss nicht notwendigerweise das komplette Messsignal 24 herangezogen werden. Es kann z.B. vorgesehen sein, dass dazu nur ein Teil des Messsignals 24 oder des verarbeiteten Messsignals 24 verwendet wird, beispielsweise der Teil, der oberhalb oder unterhalb eines bestimmten Schwellwerts liegt. Eine derartige Amplitudenmodulation ist in Fig. 13 beispielhaft dargestellt. In dem obersten Graph von Fig. 13 ist das bandpassgefilterte Messsignal 24 gegen die Zeit t aufgetragen, ferner ist der Startzeitpunkt $t_{start}$ der Stimulation angegeben. In dem mittleren Graph ist das aus dem Messsignal 24 gewonnene Modulationssignal 50 dargestellt. Zur Generierung des Modulationssignals 50 ist das Messsignal 24 nicht-linear verarbeitet worden und alle negativen Werte des Messsignals 24 bzw. des verarbeiteten Messsignals 24 sind auf Null gesetzt worden. Ferner ist das Modulationssignal 50 gegenüber dem Messsignal 24 zeitverzögert worden. Anschließend ist das so gewonnene Halbwellensignal 50 mit einer Sinusschwingungen der Frequenz $f_1$ = 1000 Hz multipliziert worden. Das Modulationssignal 50 stellt die Einhüllende der Sinusschwingung dar, wie im untersten Graph von Fig. 13 für einen kleinen Zeitausschnitt gezeigt ist. Die so gewonnene amplitudenmodulierte Sinusschwingung ist anschließend in die Stimulationseinheit 11 rückgekoppelt worden, um von dem Schallgenerator in das akustische Stimulationssignal 15 umgesetzt zu werden.

Anstelle einer Sinusschwingung mit einer einzigen Frequenz kann das Modulationssignal 50 auch mit einem beliebigen Gemisch von Sinusschwingungen (oder anderen Schwingungen) im hörbaren Frequenzbereich multipliziert werden, je nachdem, an welchen Stellen des auditorischen Cortex die Desynchronisation erfolgen soll.

Am Verlauf des in Fig. 13 dargestellten Messsignals 24 lässt sich ablesen, dass die akustische nicht-lineare zeitverzögerte Halbwellenstimulation zu einer robusten Unterdrückung der krankhaft synchronen neuronalen Aktivität führt. Der Wirkmechanismus dieser Stimulation unterscheidet sich jedoch von der Wirkungsweise des z.B. in Fig. 10 gezeigten Stimulationsverfahrens. Bei der in Fig. 13 dargestellten Stimulation wird nicht die Phase der neuronalen Aktivität in den jeweiligen stimulierten Subpopulationen zurückgesetzt, sondern die Synchronisation in der krankhaft aktiven Neuronenpopulation wird unterdrückt, indem der Sättigungsprozess der Synchronisation beeinflusst wird.

Im Folgenden wird anhand eines Beispiels erläutert, wie ein von der Messeinheit 20 gewonnenes Messsignal 24 einer nicht-linearen Prozessierung unterworfen werden kann, bevor es als Ansteuerungssignal der Stimulationseinheit 11 verwendet wird.

Ausgangspunkt ist eine Gleichung für das Ansteuerungssignal S (t) :

$$S(t) = K \cdot \overline{Z}^2(t) \cdot \overline{Z}^*(t - \tau) \tag{1}$$

In Gleichung (1) sind K ein Verstärkungsfaktor, der geeignet gewählt werden kann, und $\overline{Z}(t)$ eine mittlere Zustandsvariable des Messsignals 24. $\overline{Z}(t)$ ist eine komplexe Variable und kann folgendermaßen dargestellt werden:

$$\overline{Z}(t) = X(t) + iY(t), \qquad (2)$$

wobei $X(t)$ z.B. dem neurologischen Messsignal 24 entsprechen kann. Da die betrachteten Frequenzen im Bereich von 10 Hz = 1/100ms = $1/T_\alpha$ liegen, kann der Imaginärteil $Y(t)$ durch $X(t - \tau_\alpha)$ angenähert werden, wobei beispielsweise $\tau_\alpha = T_\alpha/4$ gilt. Damit ergibt sich:

$$S(t) = K \cdot \left[X(t) + iX(t - \tau_\alpha)\right]^2 \cdot \left[X(t - \tau) - iX(t - \tau - \tau_\alpha)\right] \qquad (3)$$

Gleichung (3) kann folgendermaßen umgeformt werden:

$$\begin{aligned} S(t) = K \cdot \big[ & X(t)^2 \cdot X(t - \tau) + i2X(t) \cdot X(t - \tau_\alpha) \cdot X(t - \tau) - X(t - \tau_\alpha) \cdot X(t - \tau) \\ & - iX(t - \tau - \tau_\alpha) \cdot X(t)^2 + 2X(t) \cdot X(t - \tau_\alpha) \cdot X(t - \tau - \tau_\alpha) \\ & + iX(t - \tau - \tau_\alpha) \cdot X(t - \tau_\alpha) \big] \end{aligned} \qquad (4)$$

Als Ansteuerungssignal für die Stimulationseinheit 11 wird der Realteil aus Gleichung (4) verwendet:

$$\text{real}[S(t)] = K \cdot \left[X(t)^2 \cdot X(t - \tau) - X(t - \tau_\alpha) \cdot X(t - \tau) + 2X(t) \cdot X(t - \tau_\alpha) \cdot X(t - \tau - \tau_\alpha)\right] \qquad (5)$$

Mit dem rückgekoppelten und eventuell weiterverarbeiteten Messsignal 24 kann der auditorische Cortex ferner gezielt an verschiedenen Stellen stimuliert werden. Im Falle von den oben beschriebenen vier verschiedenen Frequenzen $f_1$ bis $f_4$ wird das eventuell weiterverarbeitete Messsignal 24 mit einer entsprechenden Zeitverzögerung beaufschlagt und mit den Frequenzen $f_1$ bis $f_4$ multipliziert. Sofern die Stimulation weniger fokal sein soll, sondern ausgedehnter erfolgen soll, werden statt der reinen Sinusschwingungen der Frequenzen $f_1$ bis $f_4$ vier verschiedene Frequenzgemische mit den Grenzen $f_j^{unten}$ und $f_j^{oben}$ ($j = 1, 2, 3, 4$) verwendet.

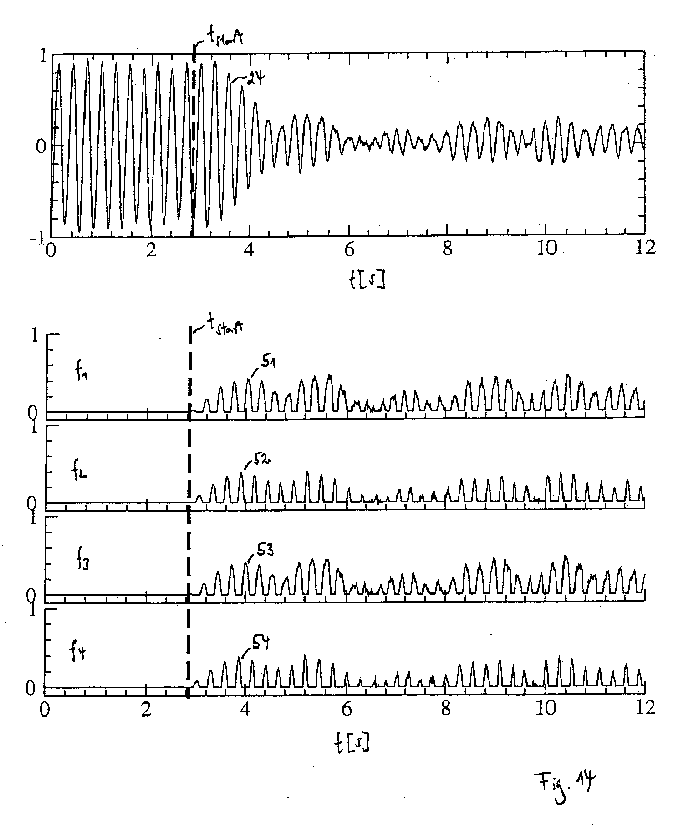

In Fig. 14 ist eine derartige Stimulation beispielhaft dargestellt. Aus dem bandpassgefilterten Messsignal 24 sind hier durch lineare Verarbeitungsschritte die Modulationssignale 51, 52, 53 und 54 gewonnen worden, mit denen Amplitudenmodulationen der Frequenzen $f_1$ bis $f_4$ durchgeführt worden sind. Durch Superposition der modulierten Sinusschwingungen ist das Steuersignal 14 erzeugt worden, welches von dem Schallgenerator 11 in das akustische Stimulationssignal 15 umgesetzt worden ist.

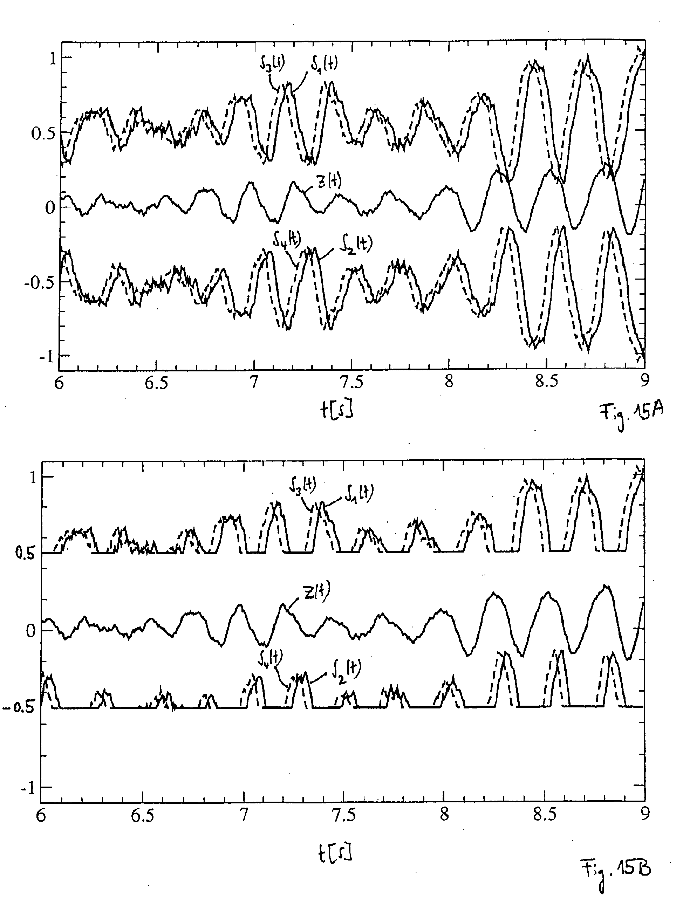

Im Folgenden wird anhand der Fig. 15A und 15B beispielhaft erläutert, wie aus dem Messsignal 24 die Modulationssignale 51 bis 54 gewonnen werden können. Dazu wird zunächst eine Verzögerungszeit $\tau$ festgelegt, die in dem vorliegenden Beispiel zu $\tau = T_{stim}/2$ gesetzt worden ist (andere Werte wie z.B. $\tau = T_{stim}$ oder $\tau = 3T_{stim}/2$ sind ebenfalls möglich). Die Frequenz $f_{stim} = 1/T_{stim}$ kann beispielsweise im Bereich der mittleren Frequenz des Messsignals 24 liegen, z.B. im Bereich von 1 bis 30 Hz, insbesondere im Bereich von 5 bis 20 Hz. Anhand der Verzögerungszeit $\tau$ können für jedes der Modulationssignale 51 bis 54 bestimmte Verzögerungszeiten $\tau_1, \tau_2, \tau_3$ und $\tau_4$ errechnet werden, beispielsweise anhand folgender Gleichung:

$$\tau_j = \tau \cdot \frac{11 - 2 \cdot (j - 1)}{8} \qquad \text{mit } j = 1, 2, 3, 4 \qquad (6)$$

Die Modulationssignale 51 bis 54 können beispielsweise aus dem Messsignal 24 gewonnen werden, indem das Messsignal 24 jeweils um die Verzögerungszeiten $\tau_1, \tau_2, \tau_3$ bzw. $\tau_4$ verzögert wird:

$$S_j(t) = K \cdot Z(t - \tau_j) \qquad\qquad (7)$$

In Gleichung (7) stehen $S_1(t)$, $S_2(t)$, $S_3(t)$ und $S_4(t)$ für die Modulationssignale 51 bis 54 und $Z(t)$ für das Messsignal 24. K ist ein Verstärkungsfaktor, der geeignet gewählt werden kann. Ferner können alle negativen Werte (oder alle Werte ober- oder unterhalb eines bestimmten Schwellwerts) der Modulationssignale $S_1(t)$ bis $S_4(t)$ auf Null gesetzt werden.

Gemäß einer in den Fig. 15A und 15B dargestellten Ausgestaltung werden die Modulationssignale $S_1(t)$ bis $S_4(t)$ nur aus den Verzögerungszeiten $\tau_1$ und $\tau_2$ errechnet, wobei die Modulationssignale $S_1(t)$ und $S_2(t)$ bzw. $S_3(t)$ und $S_4(t)$ jeweils unterschiedliche Polaritäten aufweisen:

$$S_1(t) = K \cdot Z(t - \tau_1) \qquad\qquad (8)$$

$$S_2(t) = -K \cdot Z(t - \tau_1) \qquad\qquad (9)$$

$$S_3(t) = K \cdot Z(t - \tau_2) \qquad\qquad (10)$$

$$S_4(t) = -K \cdot Z(t - \tau_2) \qquad\qquad (11)$$

Zur klareren Darstellung sind in den Fig. 15A und 15B die Modulationssignale $S_1(t)$ und $S_3(t)$ um den Wert 0,5 nach oben und die Modulationssignale $S_2(t)$ und $S_4(t)$ um den Wert 0,5 nach unten verschoben worden.

Wie in Fig. 15B gezeigt ist, können alle negativen Werte (oder alle Werte ober- oder unterhalb eines bestimmten Schwellwerts) der Modulationssignale $S_1(t)$ bis $S_4(t)$ auf Null gesetzt werden. Die Generierung der in Fig. 14 gezeigten Modulationssignale 51 bis 54 entspricht der in den Fig. 15A und 15B gezeigten Generierung der Modulationssignale $S_1(t)$ bis $S_4(t)$.

**Patentansprüche**

1. Vorrichtung (100; 400) umfassend:

    - eine Stimulationseinheit (11) zur Erzeugung eines akustischen Stimulationssignals (15), mit welchem bei einer Aufnahme über ein Ohr eines Patienten eine Population von Neuronen, die eine krankhaft synchrone und oszillatorische neuronale Aktivität aufweist, stimuliert wird, wobei
    - das akustische Stimulationssignal (15) aus N Frequenzen oder Frequenzgemischen zusammengesetzt ist, und
    - das akustische Stimulationssignal (15) eine Desynchronisation der stimulierten Neuronenpopulation bewirkt, wobei
    - jede Frequenz oder jedes Frequenzgemisch aus den N Frequenzen oder Frequenzgemischen bewirkt, dass die Phase der neuronalen Aktivität einer jeweiligen Subpopulation der stimulierten Neuronenpopulation zurückgesetzt wird,
    - die Phasen der neuronalen Aktivität der jeweiligen Subpopulationen zu unterschiedlichen Zeitpunkten zurückgesetzt werden, und
    - die N Frequenzen oder Frequenzgemische zeitversetzt kombiniert sind und der zeitliche Versatz zwischen jeweils zwei zeitlich aufeinander folgenden unterschiedlichen Frequenzen oder Frequenzgemischen im Mittel $1/(f_{stim} \times N)$ beträgt, wobei $f_{stim}$ eine Frequenz im Bereich von 1 bis 30 Hz ist, und jede der N Frequenzen oder Frequenzgemische periodisch mit der Frequenz $f_{stim}$ wiederholt wird.

2. Vorrichtung (100; 400) nach einem der vorhergehenden Ansprüche, wobei die Vorrichtung eine Steuereinheit (10) zum Ansteuern der Stimulationseinheit (11) umfasst.

**3.** Vorrichtung (400) nach einem der vorhergehenden Ansprüche, wobei die Vorrichtung eine Messeinheit (23) zum Aufnehmen eines am Patienten gemessenen Messsignals (24) umfasst.

**4.** Vorrichtung (400) nach Anspruch 3, wobei das Messsignal (24) die neuronale Aktivität im auditorischen Cortex oder einem damit verbundenen Gebiet wiedergibt.

**5.** Vorrichtung (400) nach Anspruch 3 oder 4, wobei die Steuereinheit (10) die Stimulationseinheit (11) anhand des Messsignals (24) derart ansteuert, dass die Stimulationseinheit (11) das Messsignal (24) in das akustische Stimulationssignal (15) umsetzt.

**6.** Vorrichtung (400) nach einem der Ansprüche 3 bis 5, wobei die Steuereinheit (11) das Messsignal (24) linear oder nichtlinear verarbeitet und das verarbeitete Messsignal (24) in einen Eingang der Stimulationseinheit (11) einspeist.

**7.** Vorrichtung (400) nach Anspruch 5 oder 6, wobei die Steuereinheit (10) zur Erzeugung des akustischen Stimulationssignals (15) Schwingungen mit dem Messsignal (24) oder dem verarbeiteten Messsignal (24) amplitudenmoduliert, wobei zur Amplitudenmodulation insbesondere nur ein Teil des Messsignals (24) oder des verarbeiteten Messsignals (24) herangezogen wird.

**Claims**

**1.** An apparatus (100; 400) comprising:

- a stimulation unit (11) for generating an acoustic stimulation signal (15) by which a population of neurons which has a pathologically synchronous and oscillatory neural activity on a recording via an ear of a patient is stimulated, wherein
- the acoustic stimulation signal (15) is composed of N frequencies or frequency compositions, and
- the acoustic stimulation signal (15) effects a desynchronisation of the stimulated neural population; wherein
- each frequency or each frequency composition from the N frequencies or frequency compositions has the effect that the phase of neural activity of a respective subpopulation of the stimulated neural population is reset,
- the phases of the neural activity of the respective subpopulations are reset at different times, and
- the N frequencies or frequency compositions are combined with a time offset and the time offset between two respective different frequencies or frequency compositions following one another in time amounts on average to $1/(f_{stim} \times N)$, where $f_{stim}$ is a frequency in the range from 1 to 30 Hz and each of the N frequencies or frequency compositions is periodically repeated at the frequency $f_{stim}$.

**2.** An apparatus (100; 400) apparatus in accordance with one of the preceding claims, wherein the apparatus comprises a control unit (10) for controlling the stimulation unit (11).

**3.** An apparatus (400) in accordance with one of the preceding claims, wherein the apparatus comprises a measurement unit (23) for recording a measured signal (24) measured at the patient.

**4.** An apparatus (400) in accordance with claim 3, wherein the measured signal (24) reproduces the neural activity in the auditory cortex or in a region associated therewith.

**5.** An apparatus (400) in accordance with claim 3 or claim 4, wherein the control unit (10) controls the stimulation unit (11) using the measured signal (24) such that the stimulation unit (11) converts the measured signal (24) into the acoustic stimulation signal (15).

**6.** An apparatus (400) in accordance with any one of the claims 3 to 5, wherein the control unit (11) processes the measured signal (24) in a linear or non-linear manner and feeds the processed measured signal (24) into an input of the stimulation unit (11).

**7.** An apparatus (400) in accordance with claim 5 or claim 6, wherein the control unit (10) for generating the acoustic stimulation signal (15) amplitude-modulates oscillations with the measured signal (24) or the processed measured signal (24), wherein in particular only a portion of the measured signal (24) or of the processed measured signal (24) is used.

**Revendications**

1. Dispositif (100 ; 400) comprenant :

   - une unité de stimulation (11) pour générer un signal de stimulation acoustique (15) avec lequel, lors de la réception via une oreille d'un patient, une population de neurones qui présente une activité neuronale pathologique synchrone et oscillatoire est stimulée, dans lequel
   - le signal de stimulation acoustique (15) est composé de N fréquences ou mélanges de fréquences, et
   - le signal de stimulation acoustique (15) entraîne une désynchronisation de la population de neurones stimulés, dans lequel
   - chaque fréquence ou chaque mélange de fréquences des N fréquences ou mélanges de fréquences a pour effet que la phase de l'activité neuronale d'une sous-population respective de la population de neurones stimulés est remise à zéro,
   - les phases de l'activité neuronale des sous-populations respectives sont remises à zéro à des instants différents, et
   - les N fréquences ou mélanges de fréquences sont combiné(e)s de façon décalée dans le temps et le décalage temporel entre deux fréquences différentes ou mélanges de fréquences différents mutuellement successives/successifs dans le temps s'élève en moyenne à $1/(f_{stim} \times N)$, où $f_{stim}$ est une fréquence dans la plage de 1 à 30 Hz, et chacun(e) des N fréquences ou mélanges de fréquences est répété(e) périodiquement à la fréquence $f_{stim}$.

2. Dispositif (100 ; 400) selon la revendication précédente, dans lequel le dispositif comprend une unité de commande (10) pour piloter l'unité de stimulation (11).

3. Dispositif (400) selon l'une des revendications précédentes, dans lequel le dispositif inclut une unité de mesure (23) pour recevoir un signal de mesure (24) mesuré au niveau du patient.

4. Dispositif (400) selon la revendication 3, dans lequel le signal de mesure (24) reproduit l'activité neuronale dans le cortex auditif ou une zone reliée à celui-ci.

5. Dispositif (400) selon la revendication 3 ou 4, dans lequel l'unité de commande (10) pilote l'unité de stimulation (11) au moyen du signal de mesure (24) de telle manière que l'unité de stimulation (11) convertit le signal de mesure (24) dans le signal de stimulation acoustique (15).

6. Dispositif (400) selon l'une des revendications 3 à 5, dans lequel l'unité de commande (11) traite le signal de mesure (24) de manière linéaire ou non linéaire, et le signal de mesure traité (24) est injecté dans une entrée de l'unité de stimulation (11).

7. Dispositif (400) selon la revendication 5 ou 6, dans lequel, pour engendrer le signal de stimulation acoustique (15), l'unité de commande (10) module en amplitude des oscillations avec le signal de mesure (24) ou avec le signal de mesure traité (24), dans lequel en particulier seulement une partie du signal de mesure (24) ou du signal de mesure traité (24) est utilisée pour la modulation en amplitude.

Fig. 1

Fig. 2

Fig. 3

Fig. 4

Fig. 5

600

34

35

36

31

32

30

12

33

Fig. 6

700

31

32

30

12

37

33

Fig. 7

Fig. 8

Fig. 9

Fig. 10

Fig. 11

Fig. 12

Fig. 13

Fig. 14

Fig. 15A

Fig. 15B

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10233960 A1
- US 20050049452 A1
- WO 2004098690 A1

**In der Beschreibung aufgeführte Nicht-Patentliteratur**

- **D. BILECEN ; K. SCHEFFLER ; N. SCHMID ; K. TSCHOPP ; J. SEELIG.** Tonotopic organization of the human auditory cortex as detected by BOLD-FM-RI. *Hearing Research,* 1998, vol. 126, 19-27
- **D. R. M. LANGERS ; W. H. BACKES ; P. VAN DIJK.** Representation of lateralization and tonotopy in primary versus secondary human auditory cortex. *NeuroImage,* 2007, vol. 34, 264-273
- **W. MÜHLNICKEL ; T. ELBERT ; E. TAUB ; H. FLOR.** Reorganization of auditory cortex in tinnitus. *Proc. Natl. Acad. Sci. USA,* 1998, vol. 95, 10340-10343